# EUROPÄISCHE PATENTANMELDUNG

(11) **EP 2 498 048 A1**
(43) Veröffentlichungstag der Anmeldung: **12.09.2012**
(21) Anmeldenummer: 11002000.5
(22) Anmeldetag: 10.03.2011
(51) Int. Cl.: G01B 9/02, G01N 21/47

(54) **System und Verfahren zur optischen Kohärenztomographie**

(71) Anmelder: Agfa HealthCare N.V., 2640 Mortsel (BE)
(72) Erfinder: Nebosis, Rainer, 81541 München (DE)
(74) Vertreter: Linsmeier, Josef

(57) **Zusammenfassung**

Die vorliegende Erfindung betrifft eine Vorrichtung sowie ein entsprechendes Verfahren mit einer Lichtquelle (20) zur Abgabe von Licht, einem Interferometer (10), in welches von der Lichtquelle (20) abgegebenes Licht eingekoppelt wird, zur Bestrahlung einer Probe (1) mit Licht und einem, insbesondere ortsauflösenden, Detektor (40) zur Erfassung von Interferenzmustern, die durch Überlagerung des von der Probe (1) reflektierten Lichts mit einem an einem Referenzspiegel (16) des Interferometers (10) reflektierten Teilstrahl des in das Interferometer (10) eingekoppelten Lichts erhalten wird.

Um auf möglichst einfache Weise OCT-Bilder mit möglichst hoher Auflösung und Bildqualität zu erhalten, umfasst die Lichtquelle (20) eine Strahlungsquelle (21) zur Erzeugung von räumlich inkohärentem Licht und einen optischen Filter (22) mit einer glockenförmigen oder gaußförmigen spektralen Filtercharakteristik zur Filterung des von der Strahlungsquelle (21) erzeugten Lichts.

## Beschreibung

Die vorliegende Erfindung betrifft ein System sowie ein entsprechendes Verfahren zur optischen Kohärenztomographie gemäß dem Oberbegriff der unabhängigen Ansprüche.

Die optische Kohärenztomographie (OCT) ist eine Methode, um lichtstreuende Proben in ihrem Inneren zu vermessen. Biologisches Gewebe ist aufgrund seiner lichtstreuenden Eigenschaften für die diagnostische Untersuchung mittels OCT besonders geeignet. Da die OCT mit relativ geringen Lichtintensitäten auskommt und die Wellenlängen des verwendeten Lichts zumeist in nahen Infrarotbereich (750 nm bis 1350 nm) liegen, stellt sie im Gegensatz zur ionisierenden Röntgendiagnostik für biologisches Gewebe keine Strahlenbelastung dar. Sie ist somit besonders für die Medizin von Bedeutung und grob vergleichbar mit der Ultraschalldiagnostik, wobei anstelle von Schall bei der OCT Licht verwendet wird. Die Laufzeiten des an unterschiedlichen Grenzschichten in der Probe reflektierten Lichts werden mit Hilfe eines Interferometers erfasst. Mit der OCT sind typischerweise um ein bis zwei Größenordnungen höhere Auflösungen als mit Ultraschall zu erreichen, jedoch ist die erzielbare Vermessungstiefe deutlich kleiner. Die gewonnenen Querschnittsbilder reichen aufgrund von optischer Streuung in der Regel nur bis zu einer Tiefe von wenigen Millimetern in das Gewebe hinein. Die derzeit wichtigsten Anwendungsbereiche der OCT liegen in der Ophthalmologie, der Dermatologie sowie der Krebsdiagnose. Es existieren allerdings auch einige nichtmedizinische Anwendungen, wie z.B. in der Werkstoffprüfung.

Bei OCT-Systemen werden häufig Lichtquellen mit hoher räumlicher und zeitlicher Kohärenz eingesetzt, wie z.B. Superlumineszenzdioden (SLDs). Mit dem Licht solcher Lichtquellen ist jedoch in der Regel nur eine geringe Tiefenauflösung erreichbar. Darüber hinaus können bei gleichzeitiger Verwendung eines ortsauflösenden Flächensensors aufgrund kohärenten Übersprechens sog. Geisterbilder auftreten, die nur durch eine nahezu vollständige Zerstörung der räumlichen Kohärenz vermieden werden könnten, was einerseits einen gewissen technischen Aufwand erfordert und andererseits trotz des technischen Aufwands nur bedingt möglich ist. Im Falle einer Überlagerung des Lichts mehrerer unterschiedlicher SLDs zu einer Lichtquelle können aufgrund von Nebenmaxima im Spektrum zusätzlich Geisterbilder verursacht werden. Darüber hinaus kann bei der Verwendung von SLDs aufgrund ihrer relativ geringen Leistung von bis zu etwa 20 mW, die zudem mit Erhöhung der spektralen Bandbreite abnimmt, nicht immer ein für eine hohe Bildqualität ausreichendes Signal-Rausch-Verhältnis erreicht werden.

Es ist Aufgabe der vorliegenden Erfindung, ein System sowie ein entsprechendes Verfahren zur optischen Kohärenztomographie anzugeben, mit welchem auf möglichst einfache Weise OCT-Bilder mit möglichst hoher Auflösung und Bildqualität erhalten werden.

Diese Aufgabe wird durch das System bzw. das Verfahren gemäß den unabhängigen Ansprüchen gelöst.

Das erfindungsgemäße System umfasst eine Lichtquelle zur Abgabe von Licht, ein Interferometer, in welches von der Lichtquelle abgegebenes Licht eingekoppelt wird und mit welchem eine Probe mit Licht bestrahlt werden kann, sowie einen, insbesondere ortsauflösenden, Detektor zur Erfassung von Licht, welches von der Probe reflektiert wird. Die Lichtquelle setzt sich aus einer Strahlungsquelle zur Erzeugung von räumlich inkohärentem Licht und einem optischen Filter mit einer glockenförmigen oder gaußförmigen spektralen Filtercharakteristik zur Filterung des von der Strahlungsquelle erzeugten Lichts zusammen.

Bei dem erfindungsgemäßen Verfahren wird in ein Interferometer Licht, welches von einer Lichtquelle abgegeben wird, eingekoppelt, eine Probe mit Licht, welches vom Interferometer abgegeben wird, bestrahlt und von der Probe reflektiertes Licht mit einem, insbesondere ortsauflösenden, Detektor erfasst, wobei das von der Lichtquelle abgegebene Licht erhalten wird, indem räumlich inkohärentes Licht, das von einer Strahlungsquelle erzeugt wird, mit einem optischen Filter mit einer glockenförmigen oder gaußförmigen spektralen Filtercharakteristik gefiltert wird.

Unter einer Erfassung des von der Probe reflektierten Lichts durch den Detektor ist zu verstehen, dass der Detektor Interferenzmuster erfasst, welche durch Überlagerung des von der Probe reflektierten Lichts mit einem an einem Referenzspiegel des Interferometers reflektierten Teilstrahl des in das Interferometer eingekoppelten Lichts erhalten wird. Die Überlagerung erfolgt hierbei an einem Strahlteiler des Interferometers.

Unter räumlich inkohärentem Licht im Sinne der Erfindung ist elektromagnetische Strahlung zu verstehen, deren Teilwellen zueinander keine feste räumliche Phasenbeziehung haben oder nur eine sehr kleine Kohärenzlänge aufweisen. Die Kohärenzlänge bezeichnet hierbei eine Entfernung, welche die Teilwellen zurücklegen, bis sich deren Phasenlage oder Amplitude relativ zueinander signifikant verändert hat, und ist insbesondere als der Punkt definiert, an dem der Kohärenzgrad auf 1/e abgefallen ist. Der Kohärenzgrad kann aus dem Kontrast der jeweils erhaltenen Interferenzfigur abgeleitet werden (hoher Kontrast, d.h. großer Unterschied zwischen Interferenzmaxima und -minima, entspricht einem hohem Kohärenzgrad). Eine sehr kleine Kohärenzlänge im Sinne der Erfindung ist insbesondere dann gegeben, wenn die Kohärenzlänge der von der Strahlungsquelle erzeugten elektromagnetischen Strahlung kleiner ist als 15 µm und insbesondere nur einige wenige Mikrometer beträgt.

Unter einer gaußförmigen spektralen Filtercharakteristik im Sinne der Erfindung ist zu verstehen, dass die Durchlässigkeit des optischen Filters für Licht mit bestimmten Wellenlängen λ proportional ist zu exp[-[(λ - λ₀)/2·Δλ]²], wobei λ₀ die Wellenlänge angibt, bei welcher der optische Filter seine maximale Durchlässigkeit aufweist, und Δλ die Standardabweichung bezeichnet, welche mit der Halbwertsbreite FWHM des gaußförmigen Durchlässigkeitsverlaufs wie folgt zusammenhängt: FWHM ≈ 2,35 · Δλ.

Unter einer glockenförmigen spektralen Filtercharakteristik ist ein spektraler Verlauf der Durchlässigkeit des optischen Filters zu verstehen, welcher durch einen gaußförmigen Verlauf angenähert werden kann und/oder nur soweit von einem gaußförmigen Verlauf abweicht, dass dessen Fourier-Transformierte einen im Wesentlichen gaußförmigen Verlauf mit keinen Nebenmaxima oder nur wenigen, sehr niedrigen Nebenmaxima aufweist, deren Höhe maximal 5 % des Maximums der Fourier-Transformierten beträgt.

Die Erfindung basiert auf dem Gedanken, in das Interferometer Licht einzukoppeln, das durch optische Filterung des von einer Strahlungsquelle erzeugten räumlich inkohärenten Lichts mittels eines glocken- oder gaußförmigen Filters erhalten wird.

Durch die Verwendung einer Strahlungsquelle, welche *a priori* räumlich inkohärentes Licht erzeugt, wird bei der Erfassung des von der Probe reflektierten Lichts mittels eines zweidimensionalen ortsauflösenden Detektors das Auftreten von sog. Geisterbildern aufgrund kohärenten Übersprechens von Lichtstrahlen aus unterschiedlichen Orten innerhalb der untersuchten Probe vermieden. Auf die bei einer Verwendung von räumlich kohärenten Strahlungsquellen üblicherweise erforderlichen zusätzlichen Einrichtungen zur Zerstörung der räumlichen Kohärenz kann dadurch verzichtet werden.

Darüber hinaus kann bei dem erfindungsgemäßen System bzw. Verfahren auf thermische Strahlungsquellen, wie z.B. Glüh- oder Halogenlampen, zur Erzeugung inkohärenten Lichts zurückgegriffen werden, die deutlich leistungsstärker und kostengünstiger sind als die häufig eingesetzten Superlumineszenzdioden (SLDs).

Durch die optische Filterung mit einer gauß- oder glockenförmigen Filtercharakteristik wird das von der Strahlungsquelle erzeugte Licht in zeitlich teilkohärentes Licht mit einer zeitlichen Kohärenzlänge von vorzugsweise mehr als etwa 6 µm umgewandelt. Dies ist bei dem erfindungsgemäßen System vom Typ eines sog. Time-Domain-OCT, bei welchem die Länge eines Referenzarms im Interferometer verändert und mittels eines, vorzugsweise zweidimensionalen, Detektors kontinuierlich die Intensität der auftretenden Interferenz erfasst wird, besonders vorteilhaft, da durch die Filterung des Lichts mittels des durch den optischen Filter realisierten Bandpasses einerseits eine hohe laterale Auflösung des von der Probe erfassten Bildes erreicht und durch die gauß- oder glockenförmige spektrale Filtercharakteristik des optischen Filters andererseits ein Auftreten von störenden Nebenmaxima bei der Fourier-Transformation des mit dem Detektor erfassten Interferenzmusters, welche das Auftreten weiterer Geisterbilder verursachen würden, vermieden wird.

Insgesamt werden mit dem erfindungsgemäßen System bzw. Verfahren auf einfache Weise OCT-Bilder mit hoher Auflösung und Bildqualität erhalten.

In einer ersten bevorzugten Ausführung weist der optische Filter eine maximale Durchlässigkeit für Licht auf, welche in einem Spektralbereich zwischen etwa 1200 nm und 1400 nm, insbesondere zwischen etwa 1270 nm und 1330 nm, liegt. Bei einem gaußförmigen Verlauf proportional zu exp[-[(λ - λ₀)/2·Δλ]²] liegt die Wellenlänge λ₀ in dem genannten Spektralbereich. Dadurch wird erreicht, dass das Spektrum des in das Interferometer eingekoppelte Licht ein Intensitätsmaximum im genannten Spektralbereich aufweist, was bei der Bestrahlung insbesondere von biologischem Gewebe weniger stark von der Probe absorbiert und/oder gestreut wird als Licht in anderen Spektralbereichen. Damit wird eine relative hohe Eindringtiefe erzielt.

Vorzugsweise hat der optische Filter eine Halbwertsbreite (FWHM ≈ 2,35 · Δλ), die kleiner ist als etwa 250 nm. Unter der Halbwertsbreite ist die spektrale Breite bei einer Durchlässigkeit zu verstehen, die der halben maximalen Durchlässigkeit des Filters entspricht. Bei dieser Halbwertsbreite des optischen Filters wird bei dem erfindungsgemäßen System vom Typ eines Time-Domain-OCT eine besonders hohe laterale Auflösung der von der Probe erhaltenen Bilder erreicht.

In einer bevorzugten Ausführung weist das von der Strahlungsquelle erzeugte räumlich inkohärente Licht ein Spektrum mit einer Bandbreite von mindestens 500 nm auf. Hierdurch wird gewährleistet, dass das von der Strahlungsquelle erzeugte Licht inkohärent ist bzw. eine hinreichend kleine Kohärenzlänge von vorzugsweise weniger als 15 µm aufweist. Das Auftreten von Geisterbildern bei der zweidimensionalen ortsaufgelösten Erfassung des von einem flächigen Bereich der Probe reflektierten Lichts wird dadurch mit besonders hoher Zuverlässigkeit verhindert.

Vorzugsweise weist das von der Strahlungsquelle erzeugte Licht ein kontinuierliches Spektrum auf. Dadurch wird erreicht, dass das Licht auch nach der Filterung durch den optischen Filter im Durchlässigkeitsbereich des Filters ein kontinuierliches Spektrum mit einem im Wesentlichen glatten Verlauf, insbesondere ohne Unterbrechungen, Sprünge oder dergleichen, aufweist. Dementsprechend treten bei der Fourier-Transformation der durch den Detektor erfassten Interferenzerscheinungen keine oder nur sehr schwache Nebenmaxima auf, wodurch eine besonders zuverlässige Auswertung der erfassten Interferenzerscheinungen ermöglicht wird.

Besonders vorteilhaft ist, wenn das von der Strahlungsquelle erzeugte Licht, insbesondere im Durchlässigkeitsbereich des optischen Filters, ein Spektrum ohne spektrale Linien, insbesondere ohne spektrale Emissions- und/oder Absorptionslinien, aufweist. Auch dies trägt dazu bei, dass störende Anteile in der Fourier-Transformierten der mittels des Detektors erfassten Interferenzerscheinungen stark reduziert bzw. verhindert werden.

Es ist besonders bevorzugt, dass das von der Strahlungsquelle erzeugte Licht ein Spektrum in der Form eines planckschen Strahlers, insbesondere eines schwarzen oder grauen Körpers, aufweist. Unter einem planckschen Strahler oder schwarzen Körper ist eine thermische Strahlungsquelle zu verstehen, die elektromagnetische Strahlung mit einem kontinuierlichen, charakteristischen Spektrum aussendet, das nur von der Temperatur des Strahlers abhängig ist. Ein grauer Körper hat ein Spektrum, das dem mit einem Faktor kleiner als 1 multiplizierten Spektrum des schwarzen Körpers entspricht. Der Faktor entspricht dem Emissionsgrad des grauen Körpers. Ein planckscher Strahler im Sinne der Erfindung ist aber auch ein Strahler, dessen Emissionsspektrum durch den spektralen Verlauf eines planckschen Strahlers angenähert werden kann.

Vorzugsweise weist die Strahlungsquelle eine Farbtemperatur auf, die zwischen etwa 2500 K und 3200 K, insbesondere zwischen etwa 2700 K und 3000 K, liegt. Die Farbtemperatur ist ein Vergleichswert, der den Intensitätsverlauf eines schwarzen Körpers nach dem planckschen Strahlungsgesetz und dem wienschen Verschiebungsgesetz charakterisiert. D.h. die Strahlungsquelle erzeugt Licht, dessen spektrale Zusammensetzung derjenigen eines schwarzen oder grauen Körpers mit dieser (Farb-)Temperatur entspricht. Durch die Wahl der Farbtemperatur der Strahlungsquelle im genannten Bereich wird gewährleistet, dass das erzeugte Licht ein Spektrum aufweist, das in dem bei der Untersuchung von biologischem Gewebe besonders vorteilhaften Spektralbereich, insbesondere zwischen etwa 1000 nm und 1500 nm einen relativ flachen Abfall aufweist. Insbesondere ist die mittlere Steigung des Verlaufs in diesem Spektralbereich deutlich kleiner als in anderen Spektralbereichen.

Es ist außerdem bevorzugt, dass das von der Strahlungsquelle erzeugte Licht ein Spektrum aufweist, das im betrachteten Spektralbereich höchstens einen maximalen Intensitätswert aufweist. Auch bei diesen Ausführungen wird erreicht, dass das Licht nach der Filterung durch den optischen Filter ein Spektrum mit einem im Wesentlichen glatten Verlauf, insbesondere ohne Unterbrechungen, Sprünge oder dergleichen, aufweist und annähernd die Form einer Gaußkurve aufweist. Dadurch treten bei einer Fourier-Transformation der durch den Detektor erfassten Interferenzerscheinungen keine oder allenfalls schwache Nebenmaxima auf, wodurch eine zuverlässige Auswertung der erfassten Interferenzerscheinungen ermöglicht und eine hohe Qualität des daraus abgeleiteten Bildes gewährleistet wird.

Vorteilhafterweise weist das von der Strahlungsquelle erzeugte Licht im betrachteten Spektralbereich ein Spektrum mit einem größten und einem kleinsten Intensitätswert auf, wobei die Differenz zwischen dem größten und dem kleinsten Intensitätswert höchstens 25 %, insbesondere höchstens 10 %, des größten Intensitätswertes entspricht. Alternativ oder zusätzlich weist das von der Strahlungsquelle erzeugte Licht im betrachteten Spektralbereich eine spektrale Leistungsdichte, d.h. einen Intensitätsverlauf, auf, welche nur geringfügig von der Wellenlänge abhängt oder von der Wellenlänge unabhängig ist. Durch die Wahl einer entsprechenden Strahlungsquelle bzw. des betrachteten Spektralbereichs der Strahlungsquelle wird gewährleistet, dass das erzeugte Licht ein Spektrum mit nur begrenzten maximalen Intensitätsunterschieden im betrachteten Spektralbereich aufweist, wodurch nach der Filterung des Lichts durch den optischen Filter ein relativ glatter und im Wesentlichen gauß- oder glockenförmiger Intensitätsverlauf erhalten wird, so dass auch hierdurch ein Auftreten von störenden Nebenmaxima bei der Auswertung der erfassten Interferenzerscheinungen gezielt vermindert oder sogar unterdrückt werden kann.

Bei den obengenannten Ausführungen liegt der betrachtete Spektralbereich vorzugsweise zwischen etwa 1000 nm und 1600 nm und weist eine Bandbreite von mindestens 500 nm auf. Alternativ oder zusätzlich entspricht der betrachtete Spektralbereich dem Spektralbereich, in welchem der optische Filter durchlässig ist. Alternativ oder zusätzlich liegt der Spektralbereich, in welchem der optische Filter eine maximale Durchlässigkeit für Licht aufweist, im betrachteten Spektralbereich. Die obengenannten Vorteile kommen dadurch besonders zur Geltung.

Es ist besonders bevorzugt, dass das von der Lichtquelle abgegebene Licht ein Spektrum aufweist, dessen Fourier-Transformierte ein Maximum und gegebenenfalls ein oder mehrere Nebenmaxima aufweist, deren Höhe weniger als 5 %, insbesondere weniger als 1 %, der Höhe des Maximums beträgt. Bei dieser Ausführung wird gewährleistet, dass etwaige Nebenmaxima, die bei der Auswertung der erfassten Interferenzmuster auftreten können, nur sehr schwach ausgeprägt sind und die Auswertung daher nicht oder nur unwesentlich beeinträchtigen.

Bei einer weiteren bevorzugten Ausführung wird das von der Strahlungsquelle erzeugte Licht über eine erste Fläche abgestrahlt, deren Größe in der Größenordnung der Größe einer zweiten Fläche der Probe liegt, welche mit dem vom Interferometer ausgegebenen Licht bestrahlt wird. Dies bedeutet, dass die lichtemittierende Fläche der Strahlungsquelle in etwa so groß wie - und insbesondere nicht wesentlich kleiner als - die untersuchte und mit Licht aus dem Interferometer bestrahlte Fläche auf der Probe. Alternativ oder zusätzlich gilt dies entsprechend für den Raumwinkelbereich, in welchen das von der Strahlungsquelle erzeugte Licht in Richtung des optischen Filters und des Interferometers abgegeben wird bzw. das vom Interferometer abgegebene Licht in Richtung der Probe gestrahlt wird. Durch die bezogen auf die beleuchtete Probenfläche relativ großflächige Ausgestaltung der lichtabstrahlenden Fläche bzw. des entsprechenden Raumwinkels der Strahlungsquelle wird gewährleistet, dass das von der Strahlungsquelle erzeugte räumlich inkohärente Licht seine räumliche Inkohärenz beibehält, was aufgrund des sog. Van Cittert-Zernike-Theorems bei relativ kleinen lichtabstrahlenden Flächen, wie z.B. nahezu punktförmigen Lichtquellen oder kleinformatigen Lichtquellen wie SLDs, nicht der Fall wäre. Dieser Vorteil kommt auch bei von der Strahlungsquelle erzeugtem Licht mit sehr kleiner Kohärenzlänge von insbesondere weniger als 15 µm zum tragen. Die bereits oben im Zusammenhang mit der erfindungsgemäßen Verwendung von räumlich inkohärentem Licht bzw. von Licht mit sehr kurzer Kohärenzlänge aufgeführten Vorteile werden durch diese Maßnahmen mit besonders hoher Zuverlässigkeit erreicht.

Hierbei ist bevorzugt, dass die Größen der ersten und zweiten Fläche um höchstens 20 %, insbesondere um höchstens 10 %, voneinander abweichen. Alternativ oder zusätzlich kann vorgesehen sein, dass die Größe der ersten und/oder zweiten Fläche mindestens 0,25 mm², vorzugsweise mindestens 1 mm², beträgt. Diese Maßnahmen tragen ebenfalls dazu bei, die oben genannten Vorteile aufgrund der Verwendung von inkohärentem Licht bzw. von Licht mit sehr niedriger Kohärenzlänge auf besonders einfache Weise zuverlässig zu erreichen.

Es ist außerdem vorteilhaft, wenn das von der Strahlungsquelle erzeugte Licht mit einer im Wesentlichen konstanten Intensität über eine erste Fläche abgestrahlt wird. Hierdurch wird einerseits weiter dazu beigetragen, dass das abgestrahlte Licht der Strahlungsquelle seine räumliche Inkohärenz beibehält, und andererseits eine gleichmäßige Ausleuchtung der beleuchteten Fläche auf der Probe gewährleistet.

Ein wesentlicher Aspekt des erfindungemäßen OCT-Systems betrifft also die Einschränkung der Bandbreite einer breitbandigen Strahlungsquelle mittels eines gauß- oder glockenförmigen optischen Filters. Neben den oben bereits ausführlich beschriebenen Vorteilen werden dadurch auch folgende Vorteile erzielt bzw. Probleme gelöst.

Ohne eine erfindungsgemäße Filterung des von der Strahlungsquelle erzeugten Lichts würde dieses an den zwischen Lichtquelle und Detektor befindlichen optischen Flächen (typischerweise mehr als zehn Flächen aufgrund von Linsen, Filtern, Lichtleiterfacetten, Strahlteiler etc.) mit Anti-Reflex-Beschichtungen je nach Wellenlänge unterschiedlich stark transmittiert werden und würde dort darüber hinaus in unterschiedlichen Winkeln auftreffen, was insgesamt die Homogenität der Transmission über einen großen Spektralbereich negativ beeinflusst. Im Ergebnis würde sich das Spektrum des Lichts je nach Ort im Strahlengang verändern und wäre somit undefiniert. Dies gilt insbesondere für Lichtquellen mit großer Bandbreite (FWHM größer als 300 nm), da die Anti-Reflex-Beschichtung nicht für beliebig große Bandbreiten gleichmäßig durchlässig sein kann. Entsprechende Einbußen bei der Bildqualität wären die Folge. Durch die erfindungsgemäße Filterung des von der Strahlungsquelle erzeugten inkohärenten Lichts wird das Auftreten dieser Nachteile vermieden.

Ohne die erfindungsgemäße Filterung des von der Strahlungsquelle erzeugten inkohärenten Lichts oder bei einer extrem breitbandigen Lichtquelle hätte das in das Interferometer eingekoppelte Licht extrem kurze zeitliche Kohärenzlängen (hier ist die Kohärenzlänge entlang der Ausbreitungsachse des Lichts gemeint, im Gegensatz zur räumlichen Kohärenzlänge), so dass das sog. Kohärenzgate kürzer wäre als die mittlere Wellenlänge des in das Interferometer eingekoppelten Lichts. Als Kohärenzgate wird hierbei der Abstand zweier Ebenen oder Punkte innerhalb der Probe bezeichnet, innerhalb dessen eine konstante Phasenbeziehung gegeben ist, so dass eine Interferenz auftreten kann.

Bestimmte Algorithmen zur Auswertung von Interferenzmustern wären dadurch nicht mehr funktionsfähig. Die Erfindung erlaubt dagegen die Verwendung eines digitalen Demodulationsalgorithmus, bei dem ein Interferenzmuster mit fünf Punkten in einem Abstand von π/2 abgetastet wird, ohne dass das sog. Abtasttheorem verletzt würde.

Darüber hinaus würde die weiter unten beschriebene Modulation der Lichtintensität oder Detektorempfindlichkeit zur Erhöhung der Tiefenscan-Geschwindigkeit bei zu großer Bandbreite der Lichtquelle nicht zum gewünschten Erfolg führen. Insbesondere wenn hierbei der oben beschriebene Demodulationsalgorithmus verwendet wird, bei welchem nicht die Interferenz selbst sondern eine Schwebung zwischen Interferenz und einem zweiten Signal abgetastet wird. Da die

Schwebung langwelliger ist (mindestens um den Faktor 3), ist eine Kohärenzlänge erforderlich, die größer ist als das 3,2-Fache der mittleren Wellenlänge des in das Interferometer eingekoppelten Lichts. Bei einer mittleren Wellenlänge von etwa 1300 nm bedeutet dies, dass die Kohärenzlänge größer sein muss als etwa 4 µm. Durch entsprechende Wahl der Bandbreite des optischen Filters mit gauß- oder glockenförmiger Spektralcharakteristik kann dies erreicht werden.

Weitere Vorteile, Merkmale und Anwendungsmöglichkeiten der vorliegenden Erfindung ergeben sich aus der nachfolgenden Beschreibung in Zusammenhang mit den Figuren. Es zeigen:
- Fig. 1: eine schematische Darstellung eines Systems zur optischen Kohärenztomographie;
- Fig. 2: einen Ausschnitt aus dem in Fig. 1 gezeigten System;
- Fig. 3: Beispiele zur spektralen Zusammensetzung des von der Strahlungsquelle erzeugten Lichts;
- Fig. 4: ein Vergleichsbeispiel zu den in Fig. 3 gezeigten Beispielen;
- Fig. 5: ein Beispiel zur spektralen Filtercharakteristik des optischen Filters zusammen mit den in Fig. 3 gezeigten Beispielen für die spektrale Zusammensetzung des von der Strahlungsquelle erzeugten Lichts;
- Fig. 6: ein Beispiel einer Fourier-Transformierten des Lichts der Lichtquelle; und
- Fig. 7: einen Ausschnitt aus der in Fig. 2 gezeigten Strahlungsquelle.

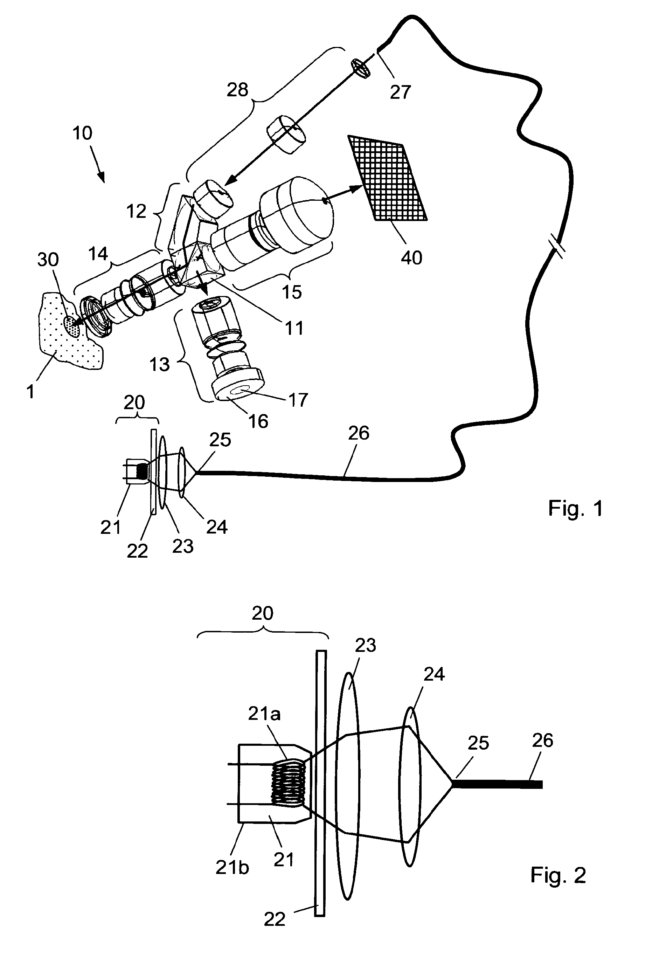

Figur 1 zeigt eine schematische Darstellung eines Systems zur optischen Kohärenztomografie mit einem Interferometer 10, welches einen Strahlteiler 11, einen Beleuchtungsarm 12, einen Referenzarm 13, einen Probenarm 14 sowie einen Detektorarm 15 umfasst. Ferner ist eine Strahlungswelle 21 zur Erzeugung von Licht vorgesehen, welches durch einen optischen Filter 22 gefiltert wird und durch eine aus Linsen 23 und 24 zusammengesetzte Optik auf einen Eingangsbereich 25 eines Lichtleiters 26 fokussiert wird. Die Strahlungsquelle 21 bildet zusammen mit dem optischen Filter 22 eine Einrichtung, welche im Zusammenhang mit der vorliegenden Erfindung auch als Lichtquelle 20 bezeichnet wird.

Das mittels der Linsen 23 und 24 in den Lichtleiter 26 eingekoppelte Licht der Strahlungsquelle 20 wird durch eine in dessen Ausgangsbereich 27 befindliche Optik 28 in den Beleuchtungsarm 12 des Interferometers 10 eingekoppelt. Von dort gelangt das eingekoppelte Licht zunächst zum Strahlteiler 11, durch welchen dieses einerseits in den Referenzarm 13 weitergeleitet und von einem an dessen Ende befindlichen beweglichen Referenzspiegel 16 reflektiert wird, und andererseits nach Durchlaufen des Probenarms 14 eine Fläche 30 einer Probe 1 beleuchtet.

Das von der Probe 1 reflektierte Licht durchläuft erneut den Probenarm 14, wird im Strahlteiler 11 mit dem am Referenzspiegel 16 reflektierten Licht aus dem Referenzarm 13 überlagert und gelangt schließlich über den Detektorarm 15 auf einen Detektor 40, der eine Vielzahl von in einer, vorzugsweise ebenen, Fläche angeordneten Detektorelemente umfasst und folglich eine ortsaufgelöste Erfassung des von der Probe 30 reflektierten Lichts bzw. eines entsprechenden Interferenzmusters aufgrund dessen Überlagerung mit dem am Referenzspiegel 16 reflektierten Licht ermöglicht.

Als Detektor 40 wird vorzugsweise eine CMOS-Kamera eingesetzt, deren Detektorelemente (sogenannte Pixel) im infraroten Spektralbereich empfindlich sind, insbesondere in einem Spektralbereich zwischen etwa 1250 nm und 1350 nm. Vorzugsweise hat die CMOS-Kamera 512 x 640 Detektorelemente.

Als Lichtleiter 26 dient vorzugsweise eine sogenannte Multimode-Faser, deren numerische Apertur und Kerndurchmesser es zulassen, dass sich bei einer bestimmten Wellenlänge des in die Faser eingekoppelten Lichts nicht nur eine Fasermode ausbilden kann, sondern viele verschiedene Fasermoden angeregt werden können. Vorzugsweise beträgt der Durchmesser der verwendeten Multimode-Faser zwischen etwa 1 mm und 3 mm, insbesondere etwa 1,5 mm.

Die Größe der beleuchteten Fläche 30 auf der Probe 1 entspricht in etwa der Größe der beleuchteten Fläche 17 auf dem Referenzspiegel 16. Die Größe der beleuchteten Fläche 30 auf der Probe 1 wird einerseits durch die am Eingangsbereich des Lichtleiters 26 befindliche Optik, welche im dargestellten Beispiel die Linsen 23 und 24 umfasst und andererseits durch die im Ausgangsbereich des Lichtleiters angeordnete Optik 28 bestimmt.

Bei dem beschriebenen System wird das entstehende Interferenzmuster mit dem Detektor 40 erfasst, wobei ein entsprechendes Interferenzsignal erzeugt wird. Die Abtastrate des Detektors 40 zum Abtasten des Interferenzsignals muss dabei so gewählt werden, dass die zeitliche Variation des Interferenzmusters mit ausreichender Genauigkeit erfasst werden kann. Dies erfordert im Allgemeinen hohe Abtastraten, wenn hohe Geschwindigkeiten für einen Tiefenscan erzielt werden sollen.

Ein Tiefenscan wird bei dem beschriebenen System vorzugsweise dadurch realisiert, dass der optische Abstand des Referenzspiegels 16 vom Strahlteiler 11 während der Erfassung des von der Probe 1 reflektierten Lichts mit dem Detektor 40 mit einer Geschwindigkeit v um einen optischen Weg verändert wird, welcher wesentlich größer ist als die mittlere Wellenlänge des in das Interferometer 10 eingekoppelten Lichts. Vorzugsweise wird hierbei das in mindestens 100 unterschiedlichen Tiefen der Probe 1 reflektierte Licht vom Detektor 40 erfasst. Es ist insbesondere bevorzugt, dass der optische Weg periodisch mit einer Amplitude verändert wird, die wesentlich größer ist als die mittlere Wellenlänge des in das Interferometer 10 eingekoppelten Lichts. Die Änderung des optischen Abstands des Referenzspiegels 16 um dem optischen Weg bzw. mit der Amplitude ist vorzugsweise mindestens 100 mal, insbesondere mindestens 1000 mal, größer ist als die mittlere Wellenlänge des in das Interferometer 10 eingekoppelten Lichts.

Da die einzelnen Perioden eines Interferenzmusters im Allgemeinen jeweils zu mehreren Zeitpunkten abgetastet werden müssen, ist die maximal mögliche Scan-Geschwindigkeit in Richtung der Tiefe der Probe 1 abhängig von der maximal möglichen Abtastrate des Detektors 40. Bei Verwendung von schnellen Detektor-Arrays mit hoher räumlicher Auflösung, d.h. großer Anzahl von Detektorelementen pro Längeneinheit, liegt die maximale Abtastrate typischerweise im Bereich von etwa 1 kHz. Dies führt bei einer mittleren Wellenlänge des in das Interferometer eingekoppelten Lichts von beispielsweise 1300 nm zu einer maximalen Geschwindigkeit für den Tiefenscan von etwa 0,1 mm/s, wenn vier Punkte pro Periode der Interferenzstruktur aufgenommen werden.

Zur Erhöhung der Geschwindigkeit des Tiefenscans wird im vorliegenden OCT-System der zeitliche Verlauf der Empfindlichkeit des Detektors 40 für das zu erfassende Licht mit einer Frequenz moduliert, die um bis zu 40 % größer oder kleiner ist als die Dopplerfrequenz f_{D}, wobei die Dopplerfrequenz f_{D} durch die mittlere Wellenlänge λ₀ des in das Interferometer 10 eingekoppelten Lichts und die Geschwindigkeit v des beweglichen Referenzspiegels 16 wie folgt gegeben ist: f_{D} = 2v/λ₀ Typische Frequenzen dieser Modulation liegen im Bereich zwischen 1 kHz und 25 kHz. Es ist besonders bevorzugt, dass die Frequenz der Modulation der Detektorempfindlichkeit ungleich der Dopplerfrequenz f_{D} ist.

Das von der Probe 1 reflektierte und auf den Detektor 40 treffende Licht überlagert sich hierbei mit der modulierten Empfindlichkeit des Detektors 40, so dass der Detektor 40 bei der Erfassung des auf den Detektor 40 treffenden Interferenzmusters anstelle eines hochfrequenten Interferenzsignals mit einer Vielzahl von Perioden ein niederfrequentes Schwebungssignal erzeugt, welches deutlich weniger Perioden aufweist als das hochfrequente Interferenzsignal. Bei der Abtastung dieser Schwebung sind daher deutlich weniger Abtastzeitpunkte pro Zeiteinheit erforderlich, ohne hierbei relevante Information zu verlieren, als bei einer Abtastung des hochfrequenten Interferenzsignals ohne die Modulation der Empfindlichkeit des Detektors 40. Bei einer gegebenen maximalen Abtastrate des Detektors 40 hat dies zur Folge, dass die maximale Geschwindigkeit für einen Tiefenscan des Systems um ein Vielfaches erhöht werden kann.

Die Empfindlichkeit des Detektors 40 lässt sich z.B. direkt oder mit einem vor dem Detektor 40 angeordneten steuerbaren elektronischen Shutter modulieren. Alternativ oder zusätzlich können Eigenschaften eines optischen Elements vor dem Detektor 40, wie z.B. die Durchlässigkeit eines Detektorobjektivs für das von der Probe 1 reflektierte Licht, moduliert werden. Im Vergleich zu Systemen mit einer konstanten Detektorempfindlichkeit wird hierdurch die Scan-Geschwindigkeit um den Faktor 4 oder sogar 8 erhöht.

Die Geschwindigkeit der Bewegung des Referenzspiegels 16 steht in einer festen Beziehung zur Frequenz der Modulation der Empfindlichkeit des Detektors 40 und ist vorzugsweise so gewählt, dass in eine Periodendauer des entstehenden Schwebungssignals eine ganzzahlige Anzahl von Abtastzeitpunkten, vorzugsweise vier Abtastzeitpunkte, passen.

Die auf diese Weise abgetasteten Schwebungssignale müssen vor einer Visualisierung noch verarbeitet werden, da in diesen Signalen noch die Interferenzinformation enthalten ist. Die wesentliche Information, die visualisiert werden soll, ist die Amplitude und Tiefenposition der jeweiligen Interferenz, nicht jedoch die Interferenzstruktur selbst. Dazu muss das Schwebungssignal demoduliert werden, indem durch Fourier-Transformation die sog. Einhüllende des Schwebungssignals bestimmt wird.

Da die Phase des Schwebungssignals im Allgemeinen unbekannt ist und diese sich auch für unterschiedliche Schwebungssignale aus unterschiedlichen Tiefen unterscheiden kann, wird ein digitaler Demodulationsalgorithmus eingesetzt, der unabhängig von der Phase ist. Vorzugsweise werden für die Abtastung des Interferenzsignals mit vier Abtastzeitpunkten pro Periode sog. 90° Phase Shift-Algorithmen verwendet. Hierdurch wird eine schnelle Demodulation des Schwebungssignals erreicht.

Vorzugsweise umfasst eine Periode der Modulation der Empfindlichkeit des Detektors 40 zwei Teilperioden, wobei während einer ersten Teilperiode der Detektor empfindlich und während einer zweiten Teilperiode der Detektor unempfindlich ist für das zu erfassende Licht. Im Allgemeinen sind die erste und die zweite Teilperiode gleich lang. Es kann jedoch von Vorteil sein, die Dauer der ersten und zweiten Teilperiode unterschiedlich lange zu wählen. Dies gilt beispielsweise dann, wenn die Intensität des von der Lichtquelle 20 abgegebenen bzw. in das Interferometer 10 eingekoppelten Lichts und/oder des von der Probe 1 reflektierten Lichts relativ gering ist. In diesen Fällen kann die erste Teilperiode so gewählt werden, dass deren Dauer länger ist als die Dauer der zweiten Teilperiode. Auf diese Weise wird auch bei geringen Lichtintensitäten neben einer hohen Tiefenscan-Geschwindigkeit ein hohes Signal-Rausch-Verhältnis und damit eine hohe Bildqualität gewährleistet.

Alternativ kann auch die Intensität des in das Interferometer 10 eingekoppelten Lichts zeitlich moduliert werden, wobei die Ausführungen zur oben beschriebenen Modulation der Detektorempfindlichkeit einschließlich der Vorteile entsprechend gelten.

Figur 2 zeigt einen Ausschnitt aus dem aus Figur 1 dargestellten System am Eingangsbereich 25 des Lichtleiters 26. Wie in der Darstellung zu erkennen ist, umfasst die Strahlungsquelle 21 einen wendelförmigen Draht 21 a, welcher von einer transparenten Umhüllung 21 b, vorzugsweise aus Glas, umgeben ist. Vorzugsweise ist die Strahlungsquelle 21 als Halogenglühlampe, insbesondere Wolfram-Halogen-Glühlampe, ausgebildet, wobei als Draht 21a ein Wolframdraht verwendet wird und das Innere der Umhüllung 21 b mit Gas gefüllt ist, welches ein Halogen, beispielsweise Jod oder Brom, enthält. Durch Anlegen einer elektrischen Spannung wird der wendelförmige Draht 21a zum Glühen gebracht, wodurch dieser räumlich inkohärentes Licht aussendet. Unter räumlich inkohärentem Licht im Sinne der vorliegenden Erfindung ist Licht zu verstehen, dessen räumliche Kohärenzlänge kleiner ist als 15 µm und insbesondere nur einige wenige µm, d.h. zwischen etwa 1 µm und 5 µm, beträgt.

Das von der Strahlungsquelle 21 erzeugte räumlich inkohärente Licht passiert den optischen Filter 22, welcher als Bandpassfilter ausgebildet ist und im Wesentlichen nur für Licht innerhalb einer vorgebbaren spektralen Bandbreite durchlässig ist. Der optische Filter 22 weist eine glockenförmige oder gausförmige spektrale Filtercharakteristik auf, wobei nur diejenigen spektralen Lichtanteile des von der Strahlungsquelle 21 erzeugten Lichts den optischen Filter 22 passieren können, welche innerhalb der vorgegebenen Bandbreite um eine mittlere Wellenlänge der glocken- bzw. gausförmigen spektralen Filtercharakteristik liegen.

Im gezeigten Beispiel ist der optische Filter 22 zwischen der Strahlungsquelle 21 und der aus den beiden Linsen 23 und 24 gebildeten eingangsseitigen Optik angeordnet. Grundsätzlich ist es aber auch möglich, den optischen Filter 22 zwischen den beiden Linsen 23 und 24 oder aber zwischen der Linse 24 und dem Eingangsbereich 25 des Lichtleiters 26 vorzusehen. Grundsätzlich ist eine Anordnung des optischen Filters 22 besonders vorteilhaft, wenn die auf den optischen Filter 22 treffenden Lichtstrahlen nur eine geringe Divergenz aufweisen oder insbesondere parallel zueinander verlaufen, da hierdurch einerseits Reflexionsverluste an den Grenzflächen des optischen Filters 22 reduziert und andererseits ein Strahlversatz aufgrund von Lichtbrechung vermindert wird. Im gezeigten Beispiel ist daher eine Anordnung des optischen Filters 22 zwischen den beiden Linsen 23 und 24 der Optik besonders bevorzugt.

Alternativ oder zusätzlich ist es aber auch möglich, den optischen Filter 22 direkt auf die Umhüllung 21 b der Strahlungsquelle 21 aufzubringen. Dies hat den Vorteil, dass auf ein zusätzliches Filterbauteil verzichtet werden kann.

Alternativ oder zusätzlich ist es aber auch möglich, den optischen Filter 22 zwischen dem Ausgangsbereich 27 des Lichtleiters 26 und dem Beleuchtungsarm 12 anzuordnen, beispielsweise vor oder zwischen den Linsen der zwischen dem Ausgangsbereich 27 des Lichtleiters 26 und dem Eingang des Beleuchtungsarms 12 befindlichen Optik 28.

Bei einer einfachen und besonders zuverlässigen Variante unfasst der optische Filter 22 einen Absorptionsfilter, insbesondere ein sogenanntes Masseglas, und einen Interferenzfilter, wobei auf das Masseglas mehrere, vorzugsweise zwischen etwa 30 und 70, dünne Schichten mit unterschiedlichen Brechungsindizes, beispielsweise durch Aufdampfen, aufgebracht werden, wodurch ein Interferenzfilter erhalten wird.

Für den Fall, dass der optische Filter 22 in die Umhüllung 21 b der Strahlungsquelle 21 integriert wird, wird der optische Filter 22 vorzugsweise durch Aufbringen solcher Interferenzschichten auf die Umhüllung 21 b realisiert. Alternativ oder zusätzlich ist es aber auch möglich, eine oder mehrere der Linsen 23, 24 bzw. der Linsen der Optik 28 mit einem entsprechenden Interferenzfilter zu versehen.

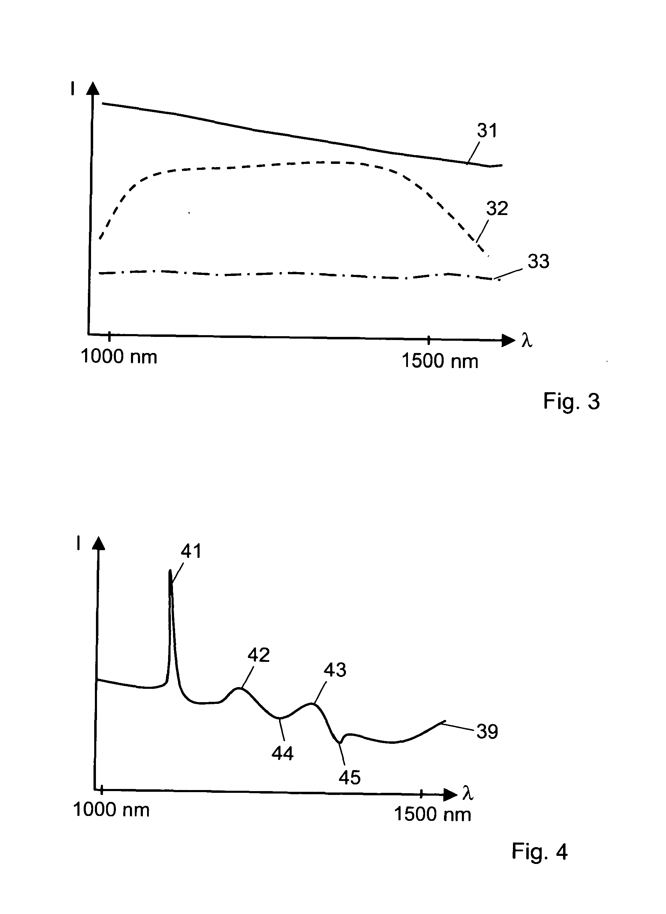

Figur 3 zeigt Beispiele zur spektralen Zusammensetzung des von der Strahlungsquelle 21 erzeugten Lichts, wobei im vorliegenden Fall die spektrale Intensität I über der Wellenlänge λ aufgetragen ist.

Der in der Figur 3 dargestellte erste Verlauf 31 entspricht einem Ausschnitt aus dem Spektrum eines Planckschen Strahlers, insbesondere eines schwarzen oder grauen Körpers, mit einer Farbtemperatur von etwa 3000 K. Der in diesem Fall betrachtete Spektralbereich liegt bei einer Wellenlänge λ zwischen etwa 1000 nm und 1500 nm. Der erste Verlauf 31 weist in diesem Spektralbereich bereits einen relativ flachen Abfall auf, so dass die Differenz zwischen der Intensität I bei einer Wellenlänge λ von etwa 1000 nm und der Intensität I bei einer Wellenlänge von etwa 1500 nm nur rund 20 % der Intensität bei einer Wellenlänge von 1000 nm entspricht.

In Figur 3 ist ferner ein zweiter spektraler Verlauf 32 des von der Strahlungsquelle 21 erzeugten Lichts dargestellt, der zwar im Bereich der oberen und unteren Grenze des betrachteten Spektralbereichs zwischen 1000 nm und 1500 nm stärker abfällt, dazwischen jedoch ebenfalls relativ flach verläuft und daher für das vorliegende System besonders geeignet ist.

Außerdem zeigt Figur 3 ein Beispiel eines dritten spektralen Verlaufs 33 des von der Lichtquelle 21 emittierten Lichts. In diesem Fall ist der Verlauf im betrachteten Spektralbereich zwischen etwa 1000 nm und 1500 nm besonders flach, so dass der Unterschied zwischen dem maximalen und dem minimalen Intensitätswert im betrachteten Spektralbereich nur wenige Prozent beträgt.

Den in den in Figur 3 dargestellten Verläufen 31, 32 und 33 ist gemein, dass diese im betrachteten Spektralbereich jeweils ein kontinuierliches Spektrum zeigen, welches keine Unterbrechungen, beispielsweise aufgrund von starken Absorptionsbanden oder dergleichen, aufweist. Darüber hinaus weisen die in Figur 3 gezeigten Spektren keine spektralen Emissionslinien auf, wie sie beispielsweise im Spektrum von Xenon-Lampen auftreten, und sie zeigen darüber hinaus einen im betrachteten Spektralbereich zwischen 1000 nm und 1500 nm im Wesentlichen glatten Verlauf ohne größere spektrale Intensitätsschwankungen.

Zum Vergleich mit den in Figur 3 gezeigten Beispielen spektraler Charakteristiken bevorzugter Strahlungsquellen 21 ist in Figur 4 beispielhaft ein spektraler Verlauf 39 des Lichts einer Strahlungsquelle dargestellt, welche im betrachteten Spektralbereich zwischen etwa 1000 nm und 1500 nm eine deutlich hervortretende spektrale Emissionslinie 41 und darüber hinaus eine starke Abhängigkeit der spektralen Intensität I von der jeweiligen Wellenlänge λ zeigt. Letzteres verdeutlichen die in der Figur dargestellten Berge 42 und 43 bzw. Täler 44 und 45.

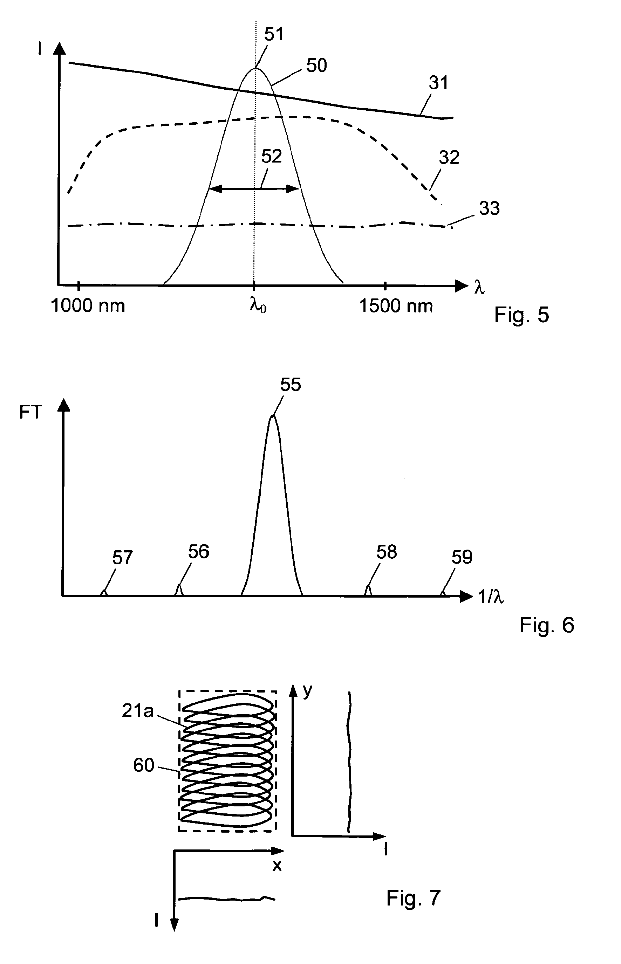

Figur 5 zeigt ein Beispiel einer spektralen Filtercharakteristik 50 des optischen Filters 22 zusammen mit den in Figur 3 gezeigten Beispielen für die spektralen Verläufe 31, 32 und 33 des von der Strahlungsquelle 21 erzeugten Lichts. Die in der Figur dargestellte spektrale Filtercharakteristik 50 hat die Form einer Glocken- oder Gaußkurve, deren Maximum 51 in einem Spektralbereich zwischen etwa 1200 nm und 1400 nm, vorzugsweise zwischen etwa 1280 nm und etwa 1320 nm, liegt und eine Halbwertsbreite 52 von weniger als etwa 250 nm aufweist.

Durch die erfindungsgemäße Filterung des von der Strahlungsquelle 21 erzeugten Lichts mit einem - im Verhältnis zur Halbwertsbreite 52 der spektralen Filtercharakteristik 50 des optischen Filters 22 von vorzugsweise weniger als 250 nm - relativ breiten Spektralbereich mit einer Breite von mehr als etwa 400 nm sowie einem relativ glatten und flachen spektralen Verlauf (siehe die beispielhaft gezeigten Verläufe 31, 32 und 33) wird Licht erhalten, dessen Spektrum einen im Wesentlichen ebenfalls glocken- oder gaußförmigen Verlauf aufweist.

Dies hat den besonderen Vorteil, dass die Fourier-Transformierte des auf diese Weise erhaltenen Lichts nur wenige Nebenmaxima aufweist, deren Höhe wesentlich geringer ist als die des Maximums der Fourier-Transformierten.

Dies wird anhand von Fig. 6 veranschaulicht, in welcher die Fourier-Transformierte FT des von der Lichtquelle 20 abgegebenen Lichts, also des von der Strahlungsquelle 21 erzeugten und durch den optischen Filter 22 gefilterten Lichts, schematisch dargestellt ist. In dem in Figur 6 gezeigten Beispiel weist die Fourier-Transformierte FT ein Maximum 55 und insgesamt vier Nebenmaxima auf, nämlich zwei Nebenmaxima 56 und 58 erster Ordnung und zwei Nebenmaxima 57 und 59 zweiter Ordnung. Wie zu erkennen ist, ist die Höhe der jeweiligen Nebenmaxima 56 bis 59 relativ zum Maximum 55 sehr klein.

Vorzugsweise beträgt die Höhe der Nebenmaxima 56 bis 59 höchstens 5 %, insbesondere weniger als 1 % der Höhe des Maximums 55. Dadurch wird das Auftreten von Geisterbildern bei der Erfassung des von der Probe 1 reflektierten und durch das Interferometer 10 hindurchtretenden Lichts mittels des Detektors 40 stark reduziert bzw. unterbunden. Hierzu trägt auch die Tatsache bei, dass das von der Strahlungsquelle 21 erzeugte Licht räumlich inkohärent ist, wodurch ein sogenanntes Übersprechen von Lichtstrahlen aus lateral unterschiedlichen Positionen in der Probe 1 verhindert wird, was andernfalls bei der Erfassung des von der Probe 1 reflektierten Lichts mittels eines zweidimensionalen, ortsauflösenden Detektors 40 ebenfalls zu Geisterbildern führen würde.

Figur 7 zeigt einen Ausschnitt im Bereich des wendelförmigen Verlaufs des Glühfadens 21a der in Figur 2 gezeigten Strahlungsquelle 21. Der wendelförmig verlaufende Glühfaden 21a der Strahlungsquelle 21 emittiert Licht in Richtung der durch die beiden Linsen 23 und 24 (siehe Fig. 2) gebildeten Optik und des Eintrittsbereichs des Lichtleiters 26, wobei der Bereich des wendelförmigen Verlaufs als erste Fläche 60 betrachtet werden kann, durch welche hindurch das von dem Glühfaden 21a erzeugte Licht in Richtung des Lichtleiters 26 abgestrahlt wird.

Wie anhand der in Figur 7 an den Seiten der ersten Fläche 60 aufgezeichneten Diagramme ersichtlich ist, ist die Intensität I des durch die erste Fläche 60 hindurchtretenden Lichts des Glühfadens 21a in beiden Dimensionen x bzw. y der ersten Fläche 60 im Wesentlichen konstant. Die Größe der ersten Fläche 60 beträgt mindestens etwa 0,25 mm², vorzugsweise mindestens 1 mm², und liegt in der Größenordnung der Größe der auf der Probe 1 beleuchteten zweiten Fläche 30 (siehe Figur 1). In diesem Größenbereich der ersten Fläche 60 bzw. bei diesem Größenverhältnis der ersten Fläche 60 zur zweiten Fläche 30 bleibt die räumliche Inkohärenz des von der Strahlungsquelle 21 erzeugten Lichts erhalten, was bei relativ kleinen Strahlungsquellen, wie z.B. nahezu punktförmigen Lichtquellen oder kleinformatigen Lichtquellen wie SLDs, nicht der Fall wäre. Die durch die erfindungsgemäße Verwendung von räumlich inkohärentem Licht bzw. von Licht mit sehr kurzer Kohärenzlänge erzielten Vorteile werden durch diese Maßnahmen mit besonders hoher Zuverlässigkeit erreicht.

## Patentansprüche

1. System zur optischen Kohärenztomographie mit
einer Lichtquelle (20) zur Abgabe von Licht,
einem Interferometer (10), in welches von der Lichtquelle (20) abgegebenes Licht eingekoppelt wird, zur Bestrahlung einer Probe (1) mit Licht und einem, insbesondere ortsauflösenden, Detektor (40) zur Erfassung von Licht, welches von der Probe (1) reflektiert wird,
**dadurch gekennzeichnet, dass**
die Lichtquelle (20) eine Strahlungsquelle (21) zur Erzeugung von räumlich inkohärentem Licht und einen optischen Filter (22) mit einer glockenförmigen oder gaußförmigen spektralen Filtercharakteristik (50) zur Filterung des von der Strahlungsquelle (21) erzeugten Lichts aufweist.

2. System nach Anspruch 1, wobei der optische Filter (22) eine maximale Durchlässigkeit (51) für Licht aufweist, welche in einem Spektralbereich zwischen etwa 1200 nm und 1400 nm, insbesondere zwischen etwa 1270 nm und 1330 nm, liegt.

3. System nach Anspruch 1 oder 2, wobei der optische Filter (22) eine Halbwertsbreite (52) aufweist, welche kleiner ist als etwa 250 nm.

4. System nach einem der vorangehenden Ansprüche, wobei das von der Strahlungsquelle (21) erzeugte Licht ein Spektrum (31, 32, 33) mit einer Bandbreite von mindestens 500 nm aufweist.

5. System nach einem der vorangehenden Ansprüche, wobei das von der Strahlungsquelle (21) erzeugte Licht ein kontinuierliches Spektrum (30, 31, 32) aufweist.

6. System nach einem der vorangehenden Ansprüche, wobei das von der Strahlungsquelle (21) erzeugte Licht ein Spektrum (31, 32, 33) ohne spektrale Linien, insbesondere ohne spektrale Emissions- und/oder Absorptionslinien, aufweist.

7. System nach einem der vorangehenden Ansprüche, wobei das von der Strahlungsquelle (21) erzeugte Licht ein Spektrum (31) in der Form eines planckschen Strahlers, insbesondere eines schwarzen oder grauen Körpers, aufweist.

8. System nach Anspruch 7, wobei die Strahlungsquelle (21) eine Farbtemperatur aufweist, welche zwischen etwa 2500 K und 3700 K, insbesondere zwischen etwa 2700 K und 3200 K, liegt.

9. System nach einem der vorangehenden Ansprüche, wobei das von der Strahlungsquelle (21) erzeugte Licht ein Spektrum (31, 32, 33) mit höchstens einem maximalen Intensitätswert, vorzugsweise in einem Spektralbereich zwischen etwa 1000 nm und 1600 nm, aufweist.

10. System nach einem der vorangehenden Ansprüche, wobei das von der Strahlungsquelle (21) erzeugte Licht ein Spektrum (31, 33) mit einem größten und einem kleinsten Intensitätswert, vorzugsweise in einem Spektralbereich zwischen etwa 1000 nm und 1600 nm, aufweist, wobei die Differenz zwischen dem größten und dem kleinsten Intensitätswert im betrachteten Spektralbereich höchstens 25 %, insbesondere höchstens 10 %, des größten Intensitätswertes entspricht.

11. System nach einem der vorangehenden Ansprüche, wobei das von der Lichtquelle (20) abgegebene Licht ein Spektrum aufweist, dessen Fourier-Transformierte (FT) ein Maximum (55) und ein oder mehrere Nebenmaxima (56 - 59) aufweist, deren Höhe weniger als 5 %, insbesondere weniger als 1 %, der Höhe des Maximums (55) beträgt.

12. System nach einem der vorangehenden Ansprüche, wobei das von der Strahlungsquelle (21) erzeugte Licht über eine erste Fläche (60) abgestrahlt wird, deren Größe in der Größenordnung der Größe einer zweiten Fläche (30) der Probe (1) liegt, welche mit dem vom Interferometer (10) ausgegebenen Licht bestrahlt wird.

13. System nach Anspruch 12, wobei die Größen der ersten und zweiten Flächen (60 bzw. 30) um höchstens 40 %, insbesondere um höchstens 25 %, voneinander abweichen.

14. System nach Anspruch 12 oder 13, wobei die Größe der ersten und/oder zweiten Fläche (60 bzw. 30) mindestens 0,25 mm², vorzugsweise mindestens 1 mm², beträgt.

15. System nach einem der vorangehenden Ansprüche, wobei das von der Strahlungsquelle (21) erzeugte Licht mit einer im Wesentlichen konstanten Intensität über eine erste Fläche (60) abgestrahlt wird.

16. Verfahren zur optischen Kohärenztomographie, bei welchem in ein Interferometer (10) Licht, welches von einer Lichtquelle (20) abgegeben wird, eingekoppelt wird,
eine Probe (1) mit Licht, welches vom Interferometer (10) abgegeben wird, bestrahlt wird und
von der Probe (1) reflektiertes Licht mit einem, insbesondere ortsauflösenden, Detektor (40) erfasst wird,
**dadurch gekennzeichnet, dass**
das von der Lichtquelle (20) abgegebene Licht erhalten wird, indem räumlich inkohärentes Licht, das von einer Strahlungsquelle (21) erzeugt wird, mit einem optischen Filter (22) mit einer glockenförmigen oder gaußförmigen spektralen Filtercharakteristik (50) gefiltert wird.
